# FASCICULE DE BREVET EUROPEEN

(11) **EP 2 981 325 B1**
(45) Date de publication et mention de la délivrance du brevet: **03.05.2017**
(21) Numéro de dépôt: 14718680.3
(22) Date de dépôt: 26.03.2014
(51) Int. Cl.: A61M 39/10, F16L 37/56

(54) **DISPOSITIF DE RACCORDEMENT POUR SYSTÈME D'ADMINISTRATION DE FLUIDES DE TRAITEMENT MÉDICAL**
KONNEKTOR FUER MEDIZINISCHES FLUID VERABREICHUNGSSYSTEM
CONNECTOR FOR MEDICAL FLUID ADMINISTRATION SYSTEM

(30) Priorité: 02.04.2013 FR 1352972
(43) Date de publication de la demande: 10.02.2016
(73) Titulaire: Doran International, 69780 Toussieu (FR)
(72) Inventeur: BUISSON, Philippe, 69780 Toussieu (FR)
(74) Mandataire: Verriest, Philippe
(86) Numéro de dépôt international: PCT/FR2014/050714
(87) Numéro de publication internationale: WO 2014/162079

(56) Documents cités:
- US-A1- 2008 105 318
- US-A1- 2012 029 479
- US-B2- 6 780 167

## Description

La présente invention concerne un dispositif de raccordement pour système d'administration de fluides de traitement médical.

Le document US 6 780 167 divulgue un dispositif de raccordement pour système d'administration de fluides de traitement médical, comprenant :
- un organe de raccordement comprenant un canal d'écoulement primaire et des canaux d'écoulement secondaires, chaque canal d'écoulement comprenant une ouverture proximale destinée à être reliée fluidiquement à un tube d'administration de fluide de traitement médical respectif et une ouverture distale, et
- un embout de connexion destiné à être connecté à un cathéter, l'embout de connexion comprenant une partie de montage dans laquelle est monté l'organe de raccordement, l'organe de raccordement et l'embout de connexion délimitant une chambre interne dans laquelle débouchent les ouvertures distales des canaux d'écoulement primaire et secondaires, l'embout de connexion comprenant en outre un orifice de sortie débouchant dans la chambre interne.

Lors de l'utilisation d'un tel dispositif de raccordement, les fluides s'écoulant à l'intérieur des canaux d'écoulement primaire et secondaires se mélangent dans la chambre interne avant d'être administrés au patient. Un tel mélange peut être désavantageux notamment lorsque les fluides issus des différents canaux d'écoulement sont incompatibles et sont susceptibles de précipiter. En effet, un mélange de tels fluides peut conduire à un traitement inadapté du patient.

Afin d'éviter un mélange des fluides issus des canaux d'écoulement primaire et secondaires, il est connu du document US 2008/0105318 de disposer l'ouverture distale du canal d'écoulement primaire entre les ouvertures distales des canaux d'écoulement secondaires, de ménager des nervures de guidage à l'intérieur de la chambre interne, et de configurer la chambre interne de telle sorte que son extrémité distale présente un diamètre supérieur à celui de son extrémité proximale.

Un tel dispositif de raccordement présente un embout de connexion complexe et nécessite une disposition spécifique des différents canaux d'écoulement. En outre, un tel dispositif de raccordement nécessite un canal d'écoulement primaire présentant une section importante afin d'éviter tout mélange des fluides issus des canaux d'écoulement secondaires, ce qui requiert un débit important du fluide s'écoulant dans le canal d'écoulement primaire.

La présente invention vise à remédier à ces inconvénients.

Le problème technique à la base de l'invention consiste notamment à fournir un dispositif de raccordement pour système d'administration de fluides de traitement médical qui soit de structure simple et économique, et qui assure une administration satisfaisante de différents fluides de traitement médical à un patient et donc un traitement satisfaisant de ce dernier.

A cet effet, la présente invention concerne un dispositif de raccordement pour système d'administration de fluides de traitement médical, comportant :
- un organe de raccordement comprenant un canal d'écoulement primaire et des canaux d'écoulement secondaires, chaque canal d'écoulement comprenant une ouverture proximale destinée à être reliée fluidiquement à un tube d'administration de fluide de traitement médical respectif et une ouverture distale, l'ouverture distale du canal d'écoulement primaire étant disposée entre les ouvertures distales des canaux d'écoulement secondaires, et
- un embout de connexion destiné à être connecté à un cathéter, l'embout de connexion comprenant une partie de montage dans laquelle est monté l'organe de raccordement, l'organe de raccordement et l'embout de connexion délimitant une chambre interne dans laquelle débouchent les ouvertures distales des canaux d'écoulement primaire et secondaires, l'embout de connexion comprenant en outre un orifice de sortie débouchant dans la chambre interne,
caractérisé en ce que la section transversale de l'ouverture distale du canal d'écoulement primaire est supérieure à la section transversale de l'ouverture proximale du canal d'écoulement primaire.

Une telle configuration du canal d'écoulement primaire, et notamment de son ouverture distale, permet, lorsque les différents canaux d'écoulement primaire et secondaire sont reliés fluidiquement à des tubes d'administration de fluides de traitement médical, de générer dans la chambre interne un flux de fluide apte à maintenir séparés les fluides issus des canaux d'écoulement secondaires jusqu'au cathéter raccordé à l'embout de connexion.

Ces dispositions permettent d'éviter un mélange de fluides incompatibles au sein de l'embout de raccordement et du cathéter, et notamment une précipitation de ces fluides incompatibles, et donc d'assurer un traitement optimal du patient.

De plus, la configuration de l'ouverture distale du canal d'écoulement primaire permet de limiter de débit du fluide s'écoulant à travers le canal d'écoulement primaire.

De préférence, le canal d'écoulement primaire est destiné à être relié à un tube d'administration connecté à un récipient contenant du sérum physiologique.

Selon un mode de réalisation de l'invention, l'ouverture distale du canal d'écoulement primaire est conformée de manière à s'étendre entre les différentes ouvertures distales des canaux d'écoulement secondaires. En d'autres termes, pour chaque canal d'écoulement secondaire, l'ouverture distale dudit canal d'écoulement secondaire est séparée des ouvertures distales des autres canaux d'écoulement secondaires par au moins une portion de l'ouverture distale du canal d'écoulement primaire.

Selon un aspect de l'invention, l'ouverture distale du canal d'écoulement primaire s'étend sur au moins 70%, et de préférence sur au moins 80%, de la largeur de la chambre interne.

Selon un mode de réalisation de l'invention, l'organe de raccordement comprend une surface d'extrémité, de préférence sensiblement plane, dans laquelle débouchent les ouvertures distales des canaux d'écoulement primaire et secondaires. De préférence, la surface d'extrémité délimite au moins en partie la chambre interne.

Selon un mode de réalisation de l'invention, l'organe de raccordement présente une forme générale tronconique.

Selon un mode de réalisation de l'invention, l'organe de raccordement comporte des moyens d'appui agencés pour coopérer avec la partie de montage de l'embout de connexion de manière à limiter la profondeur d'introduction de l'organe de raccordement dans la partie de montage. Les moyens d'appui comportent par exemple une collerette d'appui ménagée à une extrémité de l'organe de raccordement.

Selon un mode de réalisation de l'invention, la partie de montage de l'embout de connexion est sensiblement tronconique.

Selon un aspect de l'invention, chaque canal d'écoulement secondaire présente une section longitudinale constante.

Selon un aspect de l'invention, l'embout de connexion est de type Luer ou Luer Lock, et comprend une première partie de connexion tronconique reliée fluidiquement à l'orifice de sortie.

De préférence, l'embout de connexion comprend une deuxième partie de connexion filetée intérieurement et entourant la première partie de connexion tronconique.

Selon une première variante de réalisation de l'invention, la deuxième partie de connexion est montée mobile en rotation par rapport à la première partie de connexion tronconique.

Selon une deuxième variante de réalisation de l'invention, la deuxième partie de connexion est solidaire de la première partie de connexion tronconique.

Selon un mode de réalisation de l'invention, l'orifice de sortie s'étend en regard de l'ouverture distale du canal d'écoulement primaire.

Selon un aspect de l'invention, l'organe de raccordement comprend une pluralité de logements de réception dans chacun desquels est destiné à être inséré un tube d'administration de fluide de traitement médical et dans chacun desquels débouche l'ouverture proximale d'un canal d'écoulement. De préférence, les logements de réception présentent une section transversale identique et sont avantageusement sensiblement identiques.

Selon un mode de réalisation de l'invention, chaque logement de réception débouche à une extrémité de l'organe de raccordement opposée à l'embout de connexion.

Selon un aspect de l'invention, l'organe de raccordement et l'embout de connexion sont transparents.

Selon un mode de réalisation de l'invention, le canal d'écoulement primaire s'évase en direction de la chambre interne.

Selon un mode de réalisation de l'invention, les logements de réception reliés fluidiquement aux canaux d'écoulement secondaires sont répartis, de préférence régulièrement répartis, autour du logement de réception relié fluidiquement au canal d'écoulement primaire.

Selon un mode de réalisation de l'invention, les logements de réception s'étendent sensiblement parallèlement les uns par rapport aux autres. De préférence, les logements de réception s'étendent longitudinalement.

Selon un mode de réalisation de l'invention, les canaux d'écoulement secondaires s'étendent sensiblement parallèlement aux logements de réception. De préférence, chaque canal d'écoulement secondaire s'étend selon un axe sensiblement confondu avec l'axe du logement de réception respectif.

Selon un mode de réalisation de l'invention, chaque canal d'écoulement secondaire présente une section transversale circulaire.

Selon un mode de réalisation de l'invention, la chambre interne est délimitée latéralement par une paroi latérale lisse, et de préférence sensiblement cylindrique ou tronconique.

Selon un mode de réalisation de l'invention, au moins l'un des logements de réception comprend des moyens de limitation aptes à limiter la profondeur d'introduction du tube d'administration respectif. Les moyens de limitation sont de préférence formés par un épaulement.

Selon un mode de réalisation de l'invention, l'ouverture distale du canal d'écoulement primaire présente une forme oblongue, et l'organe de raccordement comprend deux canaux d'écoulement secondaires dont les ouvertures distales débouchent de part et d'autre de l'ouverture distale du canal d'écoulement primaire.

Selon un autre mode de réalisation de l'invention, l'organe de raccordement comprend quatre canaux d'écoulement secondaires et l'ouverture distale du canal d'écoulement primaire présente une forme de croix.

Selon encore un autre mode de réalisation de l'invention, l'organe de raccordement comprenant trois canaux d'écoulement secondaires et l'ouverture distale du canal d'écoulement primaire présente une forme de Y.

De toute façon l'invention sera bien comprise à l'aide de la description qui suit en référence au dessin schématique annexé représentant, à titre d'exemples non limitatifs, deux formes d'exécution de ce dispositif de raccordement.
Figure 1 est une vue éclatée en perspective d'un dispositif de raccordement selon un premier mode de réalisation de l'invention.
Figures 2 et 3 sont des vues en coupe longitudinale, en perspective, du dispositif de raccordement de la figure 1.
Figure 4 est une vue en coupe longitudinale du dispositif de raccordement de la figure 1 sur lequel sont raccordés des tubes d'administration.
Figure 5 est une vue en coupe selon la ligne V-V de la figure 4.
Figure 6 est une vue de côté d'un dispositif de raccordement selon un deuxième mode de réalisation de l'invention.
Figure 7 est une vue en coupe selon la ligne VII-VII de la figure 6.
Figure 8 est une vue en coupe selon la ligne VIII-VIII de la figure 6.

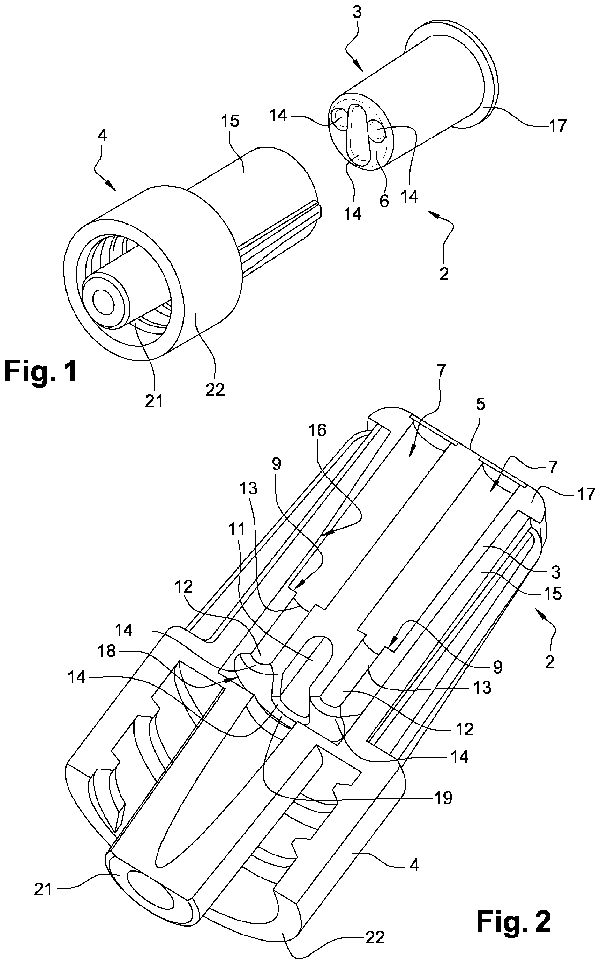

Les figures 1 à 5 représentent un dispositif de raccordement 2 pour système d'administration de fluides de traitement médical, comportant un organe de raccordement 3 destinés au raccordement de tubes d'administration de fluides de traitement médical, et un embout de connexion 4 destiné à être connecté à un cathéter. L'organe de raccordement 3 et l'embout de connexion 4 peuvent par exemple être réalisés en matière plastique transparente.

L'organe de raccordement 3 présente une forme générale tronconique et comprend une première surface d'extrémité 5 sensiblement plane opposée à l'embout de connexion 4 et une deuxième surface d'extrémité 6 sensiblement plane tournée vers l'embout de connexion 4.

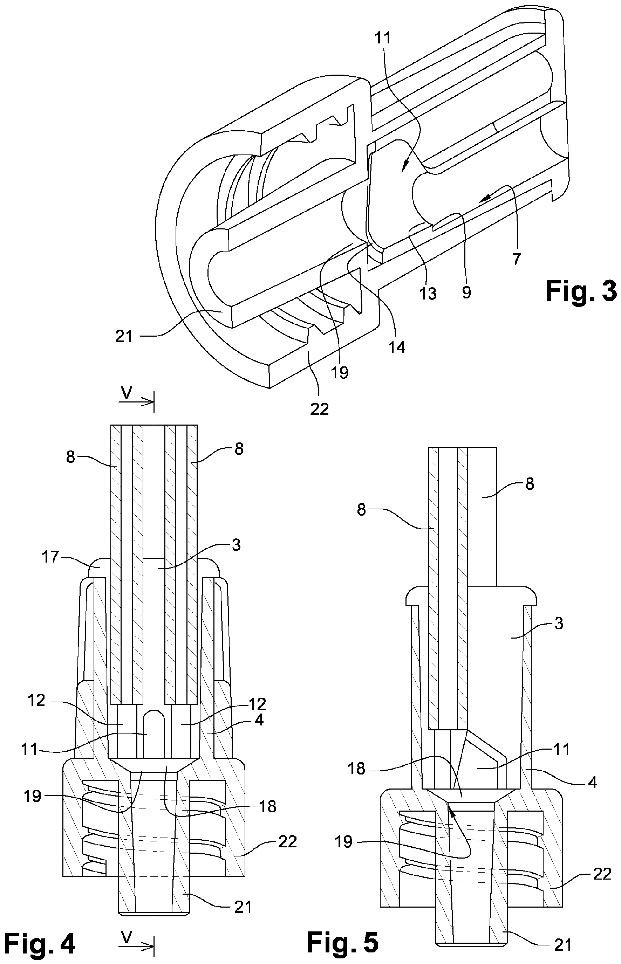

Comme montré plus particulièrement sur les figures 2 et 3, l'organe de raccordement 3 comporte une pluralité de logements de réception 7 de forme générale cylindrique débouchant dans la première surface d'extrémité 5 et s'étendant sensiblement parallèlement à l'axe longitudinal de l'organe de raccordement 3. Chaque logement de réception 7 est destiné à recevoir une portion d'extrémité d'un tube d'administration de fluide de traitement médical 8. Les différents tubes d'administration 8 peuvent par exemple être indépendants les uns des autres ou alors être reliés entre eux de manière à former un ensemble de tubes pelable.

Avantageusement, chaque logement de réception 7 comprend un épaulement 9 apte à limiter la profondeur d'introduction du tube d'administration 8 respectif dans ledit logement de réception.

L'organe de raccordement 3 comporte en outre un canal d'écoulement primaire 11 et des canaux d'écoulement secondaires 12. Chaque canal d'écoulement 11, 12 comprend une ouverture proximale 13 débouchant dans l'un des logements de réception 7 et destinée à être reliée fluidiquement au tube d'administration 8 monté dans ledit logement de réception, et une ouverture distale 14 opposée à l'ouverture proximale 13 et débouchant la deuxième surface d'extrémité 6.

Comme montré sur la figure 2, les canaux d'écoulement secondaires 12 s'étendent sensiblement parallèlement aux logements de réception 7. De préférence, chaque canal d'écoulement secondaire 12 s'étend coaxialement au logement de réception 7 correspondant.

Chaque canal d'écoulement secondaire 12 présente par exemple une section transversale circulaire et une section longitudinale constante. Le canal d'écoulement primaire 11 s'évase en direction de la deuxième surface d'extrémité 6. En particulier, la section transversale de l'ouverture distale 14 du canal d'écoulement primaire 11 est supérieure à la section transversale de l'ouverture proximale 13 du canal d'écoulement primaire 11.

Selon le mode de réalisation représenté sur les figures 1 à 5, l'ouverture distale 14 du canal d'écoulement primaire 11 présente une forme oblongue, et l'organe de raccordement 3 comprend deux canaux d'écoulement secondaires 12 dont les ouvertures distales 14 débouchent de part et d'autre de l'ouverture distale 14 du canal d'écoulement primaire 11.

L'embout de connexion 4 comprend une partie de montage 15 sensiblement tronconique délimitant un logement 16 dans lequel est monté l'organe de raccordement 3. Avantageusement, l'organe de raccordement 3 comporte une collerette d'appui 17 ménagée au niveau de la première surface d'extrémité 5 et agencée pour coopérer avec la partie de montage 15 de l'embout de connexion 4 de manière à limiter la profondeur d'introduction de l'organe de raccordement 3 dans la partie de montage 15.

L'organe de raccordement 3 et l'embout de connexion 4 délimitent une chambre interne 18 dans laquelle débouchent les ouvertures distales 14 des canaux d'écoulement primaire et secondaires 11, 12.

L'embout de connexion 4 comprend en outre un orifice de sortie 19 débouchant dans la chambre interne 18, et situé en regard de l'ouverture distale 14 du canal d'écoulement primaire 11. De préférence, l'ouverture distale 14 du canal d'écoulement primaire 11 présente une longueur correspondant à au moins 70% du diamètre de la chambre interne 18.

Selon le mode de réalisation représenté sur les figures 1 à 5, l'embout de connexion 4 est de type Luer Lock, et comprend ainsi une première partie de connexion 21 tronconique reliée fluidiquement à l'orifice de sortie 19, et une deuxième partie de connexion 22 filetée intérieurement et entourant la première partie de connexion 21. La deuxième partie de connexion 22 est solidaire de la première partie de connexion 21. Cependant, selon une variante de réalisation de l'invention, la deuxième partie de connexion 22 pourrait être montée mobile en rotation par rapport à la première partie de connexion 21. Selon encore une autre variante de réalisation de l'invention, l'embout de connexion 4 pourrait être de type Luer, et donc être dépourvu de la deuxième partie de connexion.

Le procédé d'utilisation du dispositif de raccordement 2 va maintenant être décrit.

Ce procédé d'utilisation comporte les étapes suivantes consistant à:
- connecter les première et deuxième portions de connexion 21, 22 à un cathéter (non représenté sur les figure) positionné sur un patient à traiter,
- introduire la portion d'extrémité libre d'un premier tube d'administration 8 dans le logement de réception 7 relié fluidiquement au canal d'écoulement primaire 11, le premier tube d'administration 8 étant relié fluidiquement à un premier récipient contenant par exemple du sérum physiologique,
- introduire les portions d'extrémité libres d'un deuxième et d'un troisième tubes d'administration 8 respectivement dans les logements de réception 7 reliés fluidiquement aux canaux d'écoulement secondaires 12, les deuxième et troisième tubes d'administration 8 étant reliés fluidiquement respectivement à des deuxième et troisième récipients contenant des fluides de traitement médical différents, et
- faire s'écouler les fluides contenus dans les premier, deuxième et troisième récipients dans les tubes d'administration 8 correspondants.

La configuration du canal d'écoulement primaire 11, et plus particulièrement de son ouverture distale 14, permet de générer dans la chambre interne 18 un flux de fluide apte à maintenir séparés les fluides issus des canaux d'écoulement secondaires 12 jusqu'au cathéter raccordé à l'embout de connexion 4.

Ces dispositions permettent d'éviter un mélange des fluides issus des deuxième et troisième récipients au sein de l'embout de raccordement 4 et du cathéter, et notamment une précipitation de ces fluides qui peuvent être incompatibles, et donc d'assurer un traitement optimal du patient.

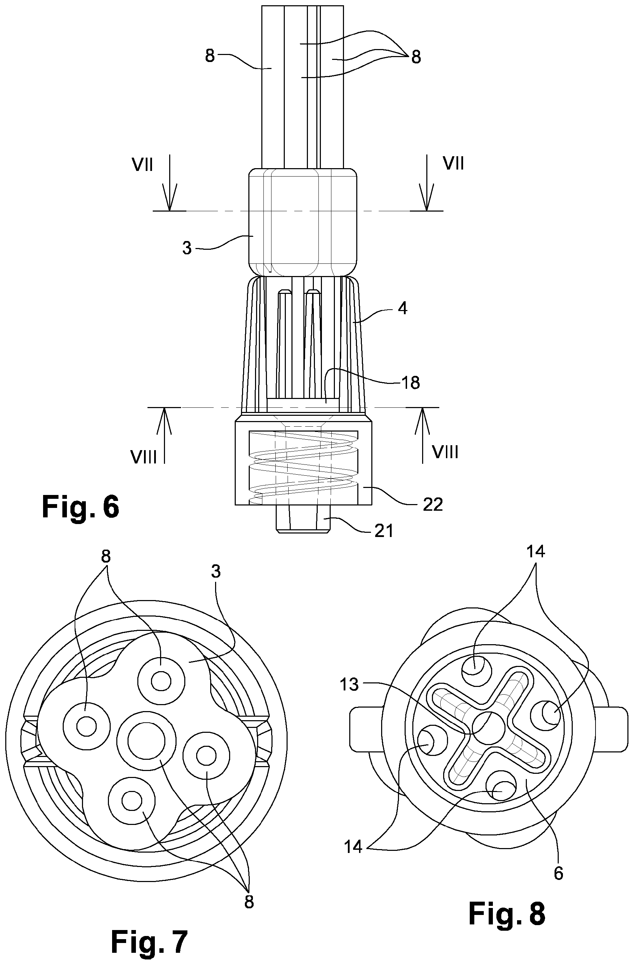

Les figures 6 à 8 représentent un dispositif de raccordement 2 selon un deuxième mode de réalisation qui diffère de celui représenté sur les figures 1 à 5 essentiellement en ce que l'organe de raccordement 3 comprend quatre canaux d'écoulement secondaires 12 et en ce que l'ouverture distale 14 du canal d'écoulement primaire 11 présente une forme de croix. Selon ce mode de réalisation, l'ouverture distale 14 du canal d'écoulement primaire 11 est conformée de manière à s'étendre entre les différentes ouvertures distales 14 des canaux d'écoulement secondaires 12. En outre, selon ce mode de réalisation, les logements de réception 7 reliés fluidiquement aux canaux d'écoulement secondaires 12 sont régulièrement répartis autour du logement de réception 7 relié fluidiquement au canal d'écoulement primaire 11.

Selon un troisième mode de réalisation de l'invention non représenté sur les figure, l'organe de raccordement 3 pourrait comprendre trois canaux d'écoulement secondaires 12 et l'ouverture distale 14 du canal d'écoulement primaire 11 pourrait présenter une forme de Y.

Comme il va de soi, l'invention ne se limite pas aux seules formes d'exécution de ce dispositif de raccordement, décrites ci-dessus à titre d'exemples, elle en embrasse au contraire toutes les variantes de réalisation.

## Revendications

1. Dispositif de raccordement (2) pour système d'administration de fluides de traitement médical, comportant :
- un organe de raccordement (3) comprenant un canal d'écoulement primaire (11) et des canaux d'écoulement secondaires (12), chaque canal d'écoulement (11, 12) comprenant une ouverture proximale (13) destinée à être reliée fluidiquement à un tube d'administration (8) de fluide de traitement médical respectif et une ouverture distale (14), l'ouverture distale (14) du canal d'écoulement primaire (11) étant disposée entre les ouvertures distales (14) des canaux d'écoulement secondaires (12), et
- un embout de connexion (4) destiné à être connecté à un cathéter, l'embout de connexion (4) comprenant une partie de montage (15) dans laquelle est monté l'organe de raccordement (3), l'organe de raccordement (3) et l'embout de connexion (4) délimitant une chambre interne (18) dans laquelle débouchent les ouvertures distales (14) des canaux d'écoulement primaire et secondaires (11, 12), l'embout de connexion (4) comprenant en outre un orifice de sortie (19) débouchant dans la chambre interne (18),
**caractérisé en ce que** la section transversale de l'ouverture distale (14) du canal d'écoulement primaire (11) est supérieure à la section transversale de l'ouverture proximale (13) du canal d'écoulement primaire (11).

2. Dispositif de raccordement selon la revendication 1, dans lequel l'ouverture distale (14) du canal d'écoulement primaire (11) est conformée de manière à s'étendre entre les différentes ouvertures distales (14) des canaux d'écoulement secondaires (12).

3. Dispositif de raccordement selon la revendication 1 ou 2, dans lequel chaque canal d'écoulement secondaire (12) présente une section longitudinale constante.

4. Dispositif de raccordement selon l'une des revendications 1 à 3, dans lequel l'embout de connexion (4) est de type Luer ou Luer Lock, et comprend une première partie de connexion tronconique (21) reliée fluidiquement à l'orifice de sortie (19).

5. Dispositif de raccordement selon la revendication 4, dans lequel l'embout de connexion (4) comprend une deuxième partie de connexion (22) filetée intérieurement et entourant la première partie de connexion tronconique (21).

6. Dispositif de raccordement selon l'une des revendications 1 à 5, dans lequel l'organe de raccordement (3) comprend une pluralité de logements de réception (7) dans chacun desquels est destiné à être inséré un tube d'administration de fluide de traitement médical (8) et dans chacun desquels débouche l'ouverture proximale (13) d'un canal d'écoulement (11, 12).

7. Dispositif de raccordement selon la revendication 6, dans lequel les logements de réception (7) présentent une section transversale identique.

8. Dispositif de raccordement selon l'une des revendications 1 à 7, dans lequel l'organe de raccordement (3) et l'embout de connexion (4) sont transparents.

9. Dispositif de raccordement selon l'une des revendications 1 à 8, dans lequel le canal d'écoulement primaire (11) s'évase en direction de la chambre interne (18).

## Patentansprüche

1. Verbindungsvorrichtung (2) für ein System zur Verabreichung von medizinischen Behandlungsfluids, umfassend:
- ein Verbindungselement (3), umfassend einen primären Ablaufkanal (11) und sekundäre Ablaufkanäle (12), wobei jeder Ablaufkanal (11, 12) eine proximale Öffnung (13) umfasst, die ausgelegt ist, um fluidisch mit einem jeweiligen Rohr zur Verabreichung (8) von medizinischem Behandlungsfluid verbunden zu sein, und eine distale Öffnung (14) umfasst, wobei die distale Öffnung (14) des primären Ablaufkanals (11) zwischen den distalen Öffnungen (14) der Ablaufkanäle (12) angeordnet ist, und
- einen Anschlussstecker (4), der ausgelegt ist, um mit einem Katheter verbunden zu sein, wobei der Anschlussstecker (4) einen Montageteil (15) umfasst, in dem das Verbindungselement (3) montiert ist, wobei das Verbindungselement (3) und der Anschlussstecker (4) eine innere Kammer (18) begrenzen, in die die distalen Öffnungen (14) der primären und sekundären Ablaufkanäle (11, 12) münden, wobei der Anschlussstecker (4) außerdem ein Ausgangsloch (19) umfasst, das in die innere Kammer (18) mündet,
**dadurch gekennzeichnet, dass** der Querschnitt der distalen Öffnung (14) des primären Ablaufkanals (11) größer als der Querschnitt der proximalen Öffnung (13) des primären Ablaufkanals (11) ist.

2. Verbindungsvorrichtung nach Anspruch 1, wobei die distale Öffnung (14) des primären Ablaufkanals (11) so angepasst ist, dass sie sich zwischen den verschiedenen distalen Öffnungen (14) der sekundären Ablaufkanäle (12) erstreckt.

3. Verbindungsvorrichtung nach Anspruch 1 oder 2, wobei jeder sekundäre Ablaufkanal (12) einen konstanten Längsschnitt aufweist.

4. Verbindungsvorrichtung nach einem der Ansprüche 1 bis 3, wobei der Anschlussstecker (4) vom Typ Luer oder Luer Lock ist und einen ersten kegelstumpfartigen Anschlussteil (21) umfasst, der fluidisch mit dem Ausgangsloch (19) verbunden ist.

5. Verbindungsvorrichtung nach Anspruch 4, wobei der Anschlussstecker (4) einen zweiten Anschlussteil (22) umfasst, der innen gewindegeschnitten ist und den ersten kegelstumpfartigen Anschlussteil (21) umgibt.

6. Verbindungsvorrichtung nach einem der Ansprüche 1 bis 5, wobei das Verbindungselement (3) eine Vielzahl von Aufnahmelagern (7) umfasst, wobei vorgesehen ist, dass in jedes dieser ein Rohr zur Verabreichung von medizinischem Behandlungsfluid (8) eingeführt wird, und wobei in jedes dieser die proximale Öffnung (13) eines Ablaufkanals (11, 12) mündet.

7. Verbindungsvorrichtung nach Anspruch 6, wobei die Aufnahmelager (7) einen identischen Querschnitt aufweisen.

8. Verbindungsvorrichtung nach einem der Ansprüche 1 bis 7, wobei das Verbindungsorgan (3) und der Anschlussstecker (4) transparent sind.

9. Verbindungsvorrichtung nach einem der Ansprüche 1 bis 8, wobei sich der primäre Ablaufkanal (11) in Richtung der inneren Kammer (18) weitet.

## Claims

1. A connecting device (2) for delivery system for medical treatment fluids, including:
- a connecting member (3) comprising a primary flow channel (11) and secondary flow channels (12), each flow channel (11,12) comprising a proximal aperture (13) intended to be fluidly connected to a respective medical treatment fluid delivery tube (8) and a distal aperture (14), the distal aperture (14) of the primary flow channel (11) being disposed between the distal apertures (14) of the secondary flow channels (12), and
- a connection end fitting (4) intended to be connected to a catheter, the connection end fitting (4) comprising a mounting portion (15) in which the connecting member (3) is mounted, the connecting member (3) and the connection end fitting (4) delimiting an inner chamber (18) in which the distal apertures (14) of the primary and secondary flow channels (11, 12) open, the connection end fitting (4) further comprising an outlet orifice (19) opening into the inner chamber (18),
**characterized in that** the cross section of the distal aperture (14) of the primary flow channel (11) is greater than the cross section of the proximal aperture (13) of the primary flow channel (11).

2. The connecting device according to claim 1, wherein the distal aperture (14) of the primary flow channel (11) is shaped to extend between the different distal apertures (14) of the secondary flow channels (12).

3. The connecting device according to claim 1 or 2, wherein each secondary flow channel (12) has a constant longitudinal section.

4. The connecting device according to any of claims 1 to 3, wherein the connection end fitting (4) is of the Luer or Luer Lock type, and comprises a first frustoconical portion (21) fluidly connected to the outlet orifice (19).

5. The connecting device according to claim 4, wherein the connection end fitting (4) comprises a second connection portion (22) internally threaded and surrounding the first frustoconical portion (21).

6. The connecting device according to any of claims 1 to 5, wherein the connecting member (3) comprises a plurality of receiving housings (7) in each of which a medical treatment fluid delivery tube (8) is intended to be inserted, and in each of which the proximal aperture (13) of a flow channel (11, 12) opens.

7. The connecting device according to claim 6, wherein the receiving housings (7) have an identical cross section.

8. The connecting device according to any of claims 1 to 7, wherein the connecting member (3) and the connection end fitting (4) are transparent.

9. The connecting device according to any of claims 1 to 8, wherein the primary flow channel (11) widens in the direction of the inner chamber (18).
